# EUROPÄISCHE PATENTSCHRIFT

(11) **EP 0 311 770 B1**
(45) Veröffentlichungstag und Bekanntmachung des Hinweises auf die Patenterteilung: **19.05.1993**
(21) Anmeldenummer: 88113210.4
(22) Anmeldetag: 16.08.1988
(51) Int. Cl.: A61M 15/00, A63B 23/00

(54) **Atemtherapiegerät**
Device for respiration therapy
Dispositif pour thérapie respiratoire

(30) Priorität: 14.08.1987 DE 3727134
(43) Veröffentlichungstag der Anmeldung: 19.04.1989
(73) Patentinhaber: Raupach, Udo, Dr., D-91477 Markt Bibart (DE)
(72) Erfinder: Raupach, Udo, Dr., D-91477 Markt Bibart (DE)
(74) Vertreter: Meyer-Roedern, Giso, Dr.

(56) Entgegenhaltungen:
- DE-U- 8 625 936
- US-A- 3 635 214
- US-A- 3 826 247
- US-A- 4 231 375
- US-A- 4 403 616

## Beschreibung

Die Erfindung betrifft ein Atemtherapiegerät für an Asthma oder spastischer Bronchitis leidende Personen mit einem Gehäuse, zu dem ein Mundstück, ein Anschluß für einen Inhalationsmittelapplikator und ein Luftausstoßkanal gehören und das eine atemstrombetätigte Ventilanordnung enthält, die beim Einatmen einen Strömungsweg von dem Mundstück in das Gehäuse freigibt und beim Ausatmen sperrt sowie beim Einatmen den Luftausstoßkanal sperrt und beim Ausatmen freigibt.

Ein solches Atemtherapiegerät ist aus dem DE-U-86 25 936 bekannt. Das Gerät hat ein Mundstück und eine Verzweigung, deren einer Zweig zu einem Dosieraerosolapplikator, und deren anderer Zweig über einen Strömungswiderstand zur Atmosphäre führt. Vor beiden Zweigen liegen atemstrombetätigte Rückschlagventile. Das Gerät erlaubt es, die beiden Therapieformen des Ausatmens gegen einen Atemwiderstand und des Einatmens aus einem Inhalator miteinander zu kombinieren. Der vom Patienten aufgebrachte Druck während des Ausatmens kann durch eine geeignete Druckmeßvorrichtung kontrolliert werden.

Aus der US-A 36 35 214 ist ein Gerät bekannt, mit dem sich das Lungenvolumen und der Volumenstrom der ausgeatmeten Luft erfassen lassen, und das in ein Inhalationsgerät umgewandelt werden kann. Das Gerät hat ein Gehäuse mit einem Mundstück und einem Steigrohr, in dem ein Kolben enthalten ist, der sich durch den Atemstrom auf und ab bewegt.

Die US-A 44 03 616 beschreibt ein Atemtherapiegerät mit einem Steigrohr, das einen Schwebekörper enthält und dessen Wand mit einer Reihe von Öffnungen versehen ist.

Ein Peak-Flow-Meter ist aus der US-A-38 26 247 bekannt.

Aufgabe der Erfindung ist es, ein Atemtherapiegerät der eingangs genannten Art zu schaffen, mit dem eine Verlangsamung der Aerosolströmung und vorteilhafte Verwirbelung des Aerosols erzielt und dem Patienten eine Kontrolle seiner Ausatmung ermöglicht wird, um insbesondere eine forcierte Ausatmung zu vermeiden und ein Ausatmen bis zum Endpunkt zu erreichen.

Diese Aufgabe wird mit einem Atemtherapiegerät der genannten Art dadurch gelöst, daß zu dem Gehäuse eine große Aerosolverwirbelungskammer mit dem Anschluß für den Inhalationsmittelapplikator und mit einem diesem Anschluß gegenüberliegenden Verwirbelungskegel gehört, und daß der Luftausstoßkanal über eine Meßund Anzeigevorrichtung für den Volumenstrom der ausgeatmeten Luft zur Atmosphäre führt, die ein Steigrohr und einen darin passend aufgenommenen Schwebekörper aufweist.

Das Steigrohr ermöglicht vorzugsweise einen Luftdurchtritt mit einem Strömungswiderstand, der mit zunehmender Höhe des Schwebekörpers abfällt. Dabei sollte über wenigstens einen Teil der Steigrohrlänge eine lineare Beziehung zwischen der Höhe des Schwebekörpers und dem Volumenstrom der austretenden Luft bestehen.

Der Schwebekörper wird vorzugsweise von einem Freikolben gebildet, z. B. in Form eines "Diaboloids". Er kann als Ventilglied fungieren und in seiner tiefsten Position das Steigrohr dicht verschließen. Damit ist auf einfache Weise sichergestellt, daß kein fehlerhaftes Einatmen durch das Steigrohr möglich ist.

Die Wand des Steigrohrs kann eine Undichtigkeit insbesondere in Gestalt einer Reihe höhenversetzter Löcher aufweisen. Von diesen Löchern werden um so mehr freigegeben, je höher der Schwebekörper in dem Steigrohr steht. Man kann die Anzahl und Größe der Löcher leicht so wählen, daß der Schwebekörper eine genaue Anzeige des ausgeatmeten Volumenstroms liefert und die gewünschte lineare Anzeigebeziehung zwischen Volumenstrom und der Höhe des Schwebekörpers besteht. Ähnliches gilt für eine Undichtigkeit in Gestalt eines Schlitzes, der sich in Steigrohrlängsrichtung erstreckt.

Bei einer bevorzugten Ausführungsform des Atemtherapiegeräts ist eine verstellbare Abdeckung vorgesehen, mit der sich die Undichtigkeit zum Teil verschließen läßt. Die Abdeckung erlaubt es, eine Anpassung der Anzeige an das Lungenvolumen des Patienten vorzunehmen, das ebenso wie die eingentlich interessierende Strömungsgeschwindigkeit der Ausatmung in den gemessenen Volumenstrom eingeht. In einer bevorzugten Bauform wird die Abdeckung von einer drehbar auf das Steigrohr aufgezogenen Überwurfhülse gebildet. Die Überwurfhülse kann einen Ausschnitt haben, dank dessen sie je nach Winkelstellung eine mehr oder weniger große Anzahl von Löchern bzw. eine mehr oder weniger lange Partie des Schlitzes abdeckt. Alternativ ist die Überwurfhülse mit mehreren Reihen untereinander vorzugsweise gleich großer, von Reihe zu Reihe verschieden großer Drosselbohrungen versehen, die je nach Winkelstellung der Überwurfhülse mit der Lochreihe des Steigrohrs fluchten.

Das Steigrohr kann unter einem Winkel zwischen 0° und 20°, vorzugsweise ca. 10°, gegen die Vertikale angestellt sein. Bei dieser Anordnung liegt der Schwebekörper gut im Blickfeld des Patienten, und es ist eine parallaxenfreie Ablesung möglich.

Das Steigrohr kann lösbar mit dem Gehäuse verbunden und gegen ein zur Peak-Flow-Messung dienendes Aufsatzrohr austauschbar sein. Dieses enthält vorzugsweise einen Kolben und ein reibschlüssig in dem Aufsatzrohr geführtes, selbsthemmend daran festliegendes, von dem Kolben mitgenommenes Anzeigeelement. Mit diesem wird der erreichte Spitzenwert des Volumenstroms auf Dauer angezeigt. In einer bevorzugten Bauform ist der Kolben des Aufsatzrohrs auf eine Führungsstange aufgezogen und durch eine Feder niedergespannt. Als Anzeigeelement kann eine längsgeschlitzte elastische Hülse dienen. Bei einer Undichtigkeit des Aufsatzrohrs in Gestalt eines Längsschlitzes besteht die Möglichkeit, das Anzeigeelement mit einem durch den Schlitz hindurchragenden Griffansatz zu versehen, mit dem es von Hand bewegt und insbesondere an den Fuß des Aufsatzrohrs zurückgestellt werden kann.

Die Erfindung wird im folgenden anhand von in den Zeichnungen dargestellten Ausführungsbeispielen näher erläutert. Es zeigen:
- Fig. 1: die Seitenansicht eines ersten Atemtherapiegeräts;
- Fig. 2: eine Vorderansicht des Atemtherapiegeräts in Blickrichtung II eines Benutzers;
- Fig. 3: die alternative Bauform einer auf dem Steigrohr des Atemtherapiegeräts sitzenden Überwurfhülse;
- Fig. 4: eine Mantelabwicklung der Überwurfhülse gemäß Fig. 3;
- Fig. 5: die Fig. 1 entsprechende Seitenansicht eines zweiten Atemtherapiegeräts mit einem Aufsatzrohr zur Peak-Flow-Messung,das wahlweise mit dem zur Atemtherapie dienenden Steigrohr auswechselbar ist;
- Fig. 6: einen Querschnitt durch das Aufsatzrohr nach VI - VI von Fig. 5;
- Fig. 7: als Einzelheit einen in dem Steigrohr aufgenommenen, diaboloidförmigen Schwebekörper;
- Fig. 8: in Seitenansicht ein Ventil des Atemtherapiegeräts;
- Fig. 9: eine Draufsicht auf das Ventil mit Blick in Richtung IX von Fig. 8;
- Fig. 10: eine Draufsicht auf das zugehörige Ventilglied mit Blick in Richtung X von Fig. 8; und
- Fig. 11: die schematische Seitenansicht eines dritten Atemtherapiegeräts.

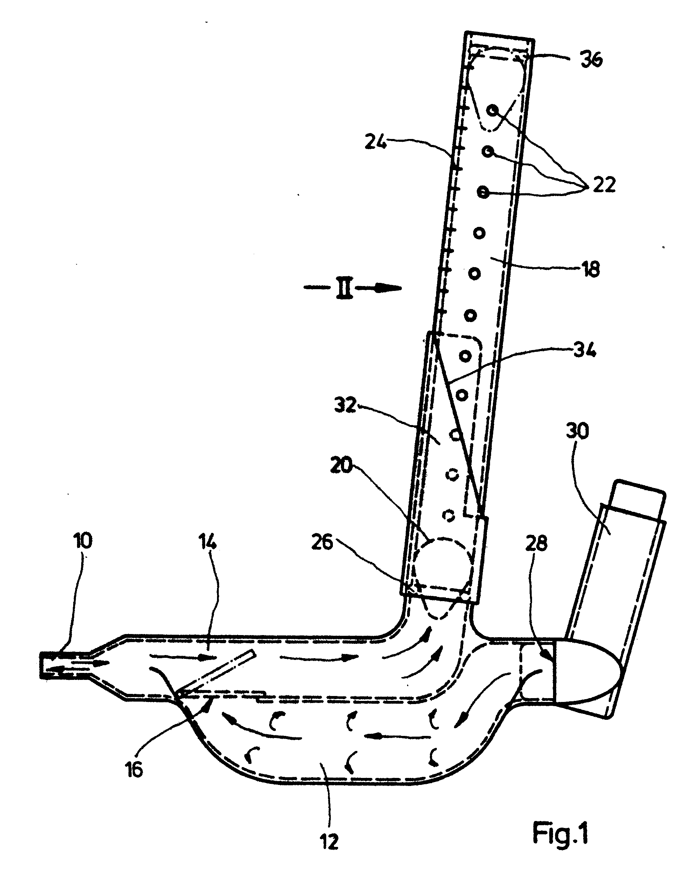

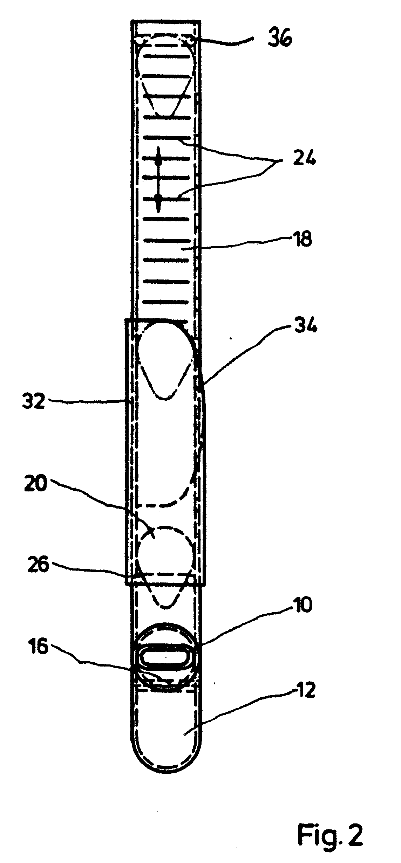

Das Atemtherapiegerät gemäß Fig. 1 und 2 hat ein Gehäuse, das sich hinter einem Mundstück 10 in einen Luftansaugkanal 12 und einen Luftausstoßkanal 14 verzweigt. Der Luftausstoßkanal 14 bildet eine geradlinige Verlängerung des Mundstücks 10, während der Luftansaugkanal 12 nach unten davon abgeht und sich unmittelbar an den Luftausstoßkanal 14 angrenzend abschnittsweise parallel dazu erstreckt. Die Öffnung des Luftansaugkanals 12 am Abzeig ist durch ein Klappenventil verschlossen, dessen Ventilklappe 16 beim Einatmen durch das Mundstück 10 öffnet und beim Ausatmen schließt.

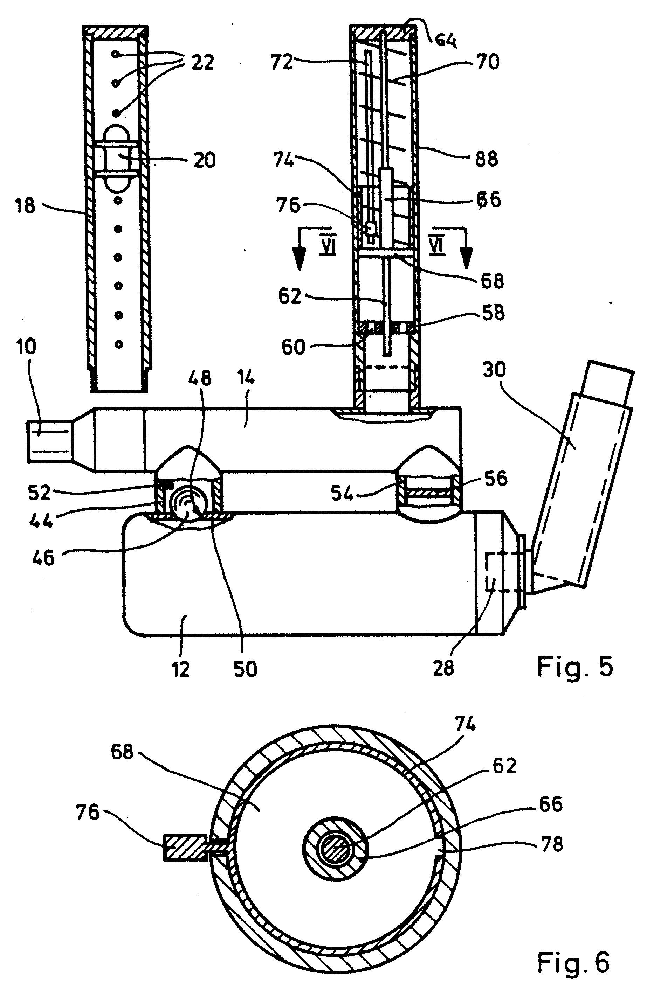

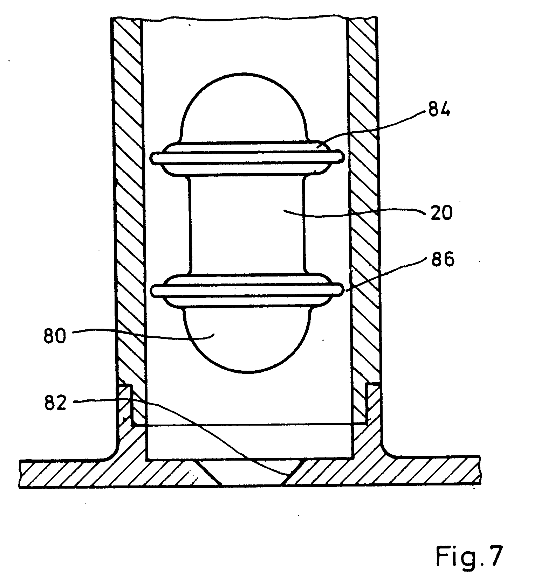

Der Luftausstoßkanal 14 geht in ein ebenfalls gerades Steigrohr 18 über, das unter einem Winkel von ca. 100° nach oben davon abgewinkelt ist. Das Steigrohr 18 enthält einen Schwebekörper 20 beispielsweise in Gestalt eines kugel- oder ballonförmigen oder als Diaboloid (vgl. Fig. 5 und 7) gestalteten Hohl- oder Massivkörpers von geringem Gewicht.

In der Wand des Steigrohrs 18 sind eine Anzahl Luftlöcher 22 vorgesehen, die bei dem dargestellten Ausführungsbeispiel in einer Reihe und unter gleichem Abstand angeordnet sind. Die Löcher 22 haben im wesentlichen gleiche Größe. Es versteht sich aber, daß die Wand des Steigrohrs 18 auch in anderer Weise mit einer Undichtigkeit versehen sein kann, derer Luftdurchtrittsquerschnitt umso größer ist, je höher der Schwebekörper 20 in dem Steigrohr 18 steht. Der Schwebekörper 20 gleitet als Freikolben mit Spiel und unter geringer Reibung in dem Steigrohr 18, wobei um ihn herum nur in einem geringen, wohldefinierten Maß Fehlluft auftritt. Je höher der Schwebekörper 20 beim Ausatmen geblasen wird, desto mehr Löcher 22 werden für den Luftdurchtritt freigegeben. Die Höhenposition des Schwebekörpers 20 bildet damit ein Maß für den Volumenstrom der ausgeatmeten Luft und ist diesem über wenigstens einen Teil der Steigrohrlänge linear proportional.

Das Gewicht des Schwebekörpers 20 darf nicht zu gering sein. Das Gewicht ergibt den "primären Atemwiderstand", d. h. daß beim Hochsteigen des Diaboloids die Ausatemluft durch die freiwerdenden Löcher auch mit einem gewissen Druck abgeblasen wird.

Das Steigrohr 18 kann an seinem Ende offen sein, sollte dort aber einen Anschlag 36 für den Schwebekörper 20 bilden, so daß dieser darin gefangen ist. Vorzugsweise ist das Steigrohr 18 aber am Ende verschlossen, damit beim raschen Hochblasen des Diaboloids sich ein federndes Luftpolster zwischen dem letzten Loch und dem Deckel des Steigrohrs 18 bildet.

Das Steigrohr 18 besteht aus durchsichtigem Kunststoff, so daß der Benutzer den Schwebekörper 20 beobachten kann, während er durch das Mundstück 10 atmet. Die leichte Neigung des Steigrohrs 18 vom Benutzer weg ist im Hinblick auf gute Sichtverhältnisse günstig. Eine vorzugsweise farbige Skala 24 auf dem Steigrohr 18 ermöglicht es dem Benutzer, sein Ausatmen zu bewerten. Bei günstigen Strömungsverhältnissen bleibt der Schwebekörper 20 in einem niedrigen oder mittleren Bereich des Steigrohrs 18, während er bei forciertem, stoßartigem Ausatmen bis in den oberen Bereich des Steigrohrs 18 gelangt.

Am unteren Ende des Steigrohrs 18 ist ein Ventilsitz 26 ausgebildet, mit dem der Schwebekörper 20 in seiner niedrigsten Stellung als Ventilglied zusammenarbeitet. Das Steigrohr 18 ist dadurch abgesperrt, so daß kein Einatmen dadurch möglich ist.

Der Luftansaugkanal 12 folgt abschnittsweise der Krümmung des Knies, das von dem Luftausstoßkanal 14 und dem Steigrohr 18 gebildet wird, und läuft dann im wesentlichen in axialer Verlängerung von Mundstück 10 und Luftausstoßkanal 14 aus. Die Mündung 28 des Luftansaugkanals 12 bildet eine Öffnung zur Atmosphäre, die einerseits ein unmittelbares Einatmen von Luft, und andererseits den Anschluß eines Inhalationsmittelapplikators ermöglicht. Bei dem dargestellten Ausführungsbeispiel ist insofern ein Dosieraerosolapplikator 30 vorgesehen, der in die Mündung 28 des Luftansaugkanals 12 eingesteckt wird. Es versteht sich aber, daß auch ein Anschluß anderer Inhalationsgeräte möglich ist und ganz allgemein Gase, Dämpfe und versprühte Flüssigkeiten zu therapeutischen Zwecken oder Testzwecken inhaliert werden können. Der Luftansaugkanal 12 bildet dabei eine Pufferkammer, aus der über das druckabhängig schaltende Klappenventil eingeatmet wird, sobald im Mundstück 10 ein Unterdruck herrscht. Der Benutzer braucht daher die Zudosierung/des Inhalats, die beispielsweise durch Auslösen eines Sprühstoßes an dem Dosieraerosolapplikator 30 erfolgt, nicht mit dem Gang seiner Atmung zeitlich exakt zu koordinieren.

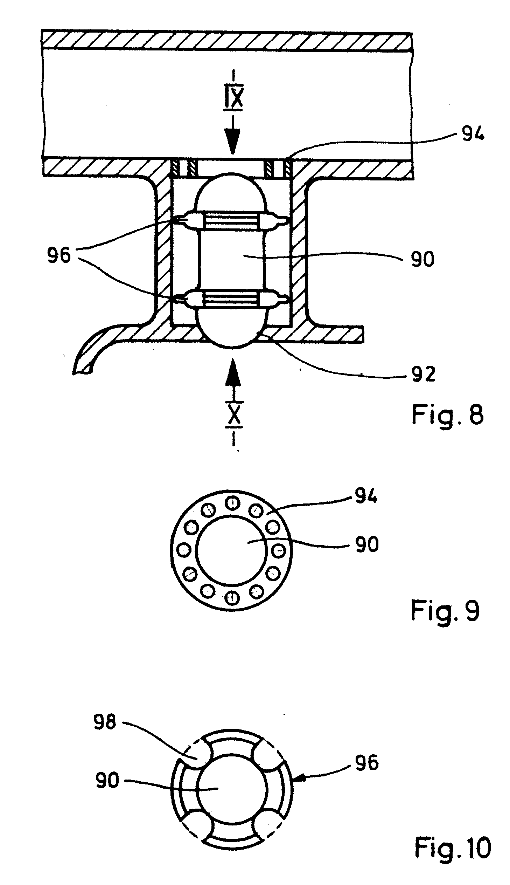

Als Ventil zwischen Luftansaugkanal 12 und Luftausstoßkanal 14 kommt neben dem beschriebenen Klappenventil und einem Kugelventil, das leicht flattert, ein Halbkugelventil mit zylindrischer Führung in Betracht, das in Fig. 8 bis 10 dargestellt ist. Das Ventilglied 90 ist ein zur Erleichterung des Einbaus symmetrisch aufgebauter, ähnlich wie der Schwebekörper 20 in dem Steigrohr 18 gestalteter Diaboloid. Das Ventilglied 90 arbeitet einends mit einem Ventilsitz 92 zusammen und ist andernends durch eine Anschlagnase bzw. einen gelochten Ringsitz 94 gehalten. In den Diaboloidringen 96 befinden sich Aussparungen 98 für den Übertritt des einzuatmenden Aerosolgemischs.

Der an dem Steigrohr 18 angezeigte Volumenstrom der ausgeatmeten Luft hängt von dem Lungenvolumen des Patienten und der eigentlich interessierenden Strömungsgeschwindigkeit der Ausatmung gleichermaßen ab. Um die unterschiedlichen Lungenvolumina verschiedener Patienten zu kompensieren und eine vergleichbare Anzeige zu erhalten, ist auf dem unteren Teil des Steigrohrs 18 eine Überwurfhülse 32 aufgesetzt, deren Mantel einen Ausschnitt 34 aufweist. Die Überwurfhülse 32 läßt sich auf dem Steigrohr 18 drehen, wobei sie je nach Winkelstellung eine mehr oder weniger große Anzahl von Löchern 22 abdeckt. Die mit dem Schwebekörper 20 erfolgende Anzeige wird so in einstellbarer Weise verschoben, und zwar durch Abdecken von Löchern 22 nach oben und durch Freigeben von Löchern 22 nach unten. Die Überwurfhülse 32 ist vorzugsweise durchsichtig. Der Rand ihres Ausschnitts 34 kann eine Schraubenlinie bilden; die Basis kann ein Rändelring sein.

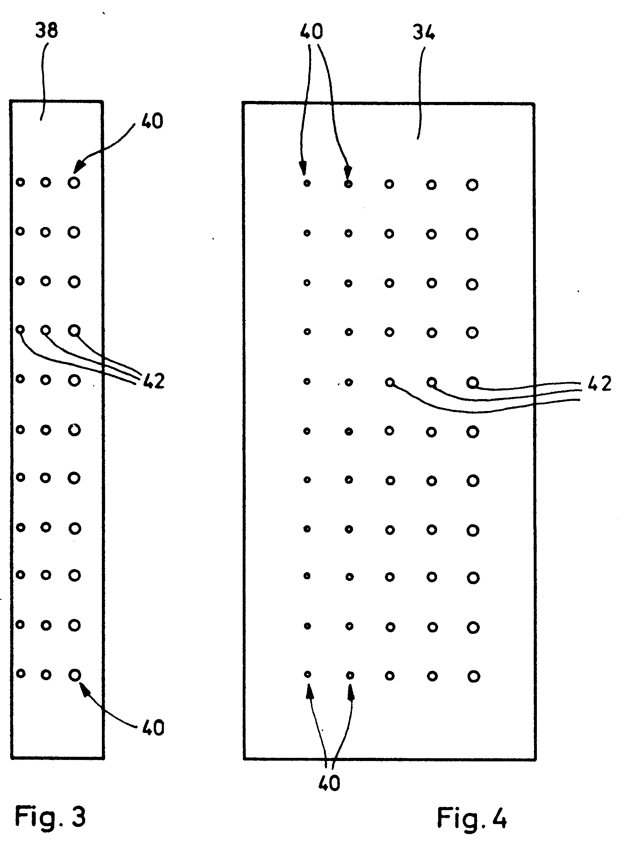

Fig. 3 und 4 zeigen eine alternative Überwurfhülse, die sich über die volle Länge des Steigrohrs 18 erstreckt und mit mehreren, in Längsrichtung verlaufenden Reihen 40 von Drosselbohrungen 42 versehen ist. Der Abstand der Drosselbohrungen einer Reihe 40 entspricht dem der Löcher 22 im Steigrohr 18, so daß je nach Winkelstellung der Überwurfhülse 38 die eine oder andere Reihe 40 von Drosselbohrungen 42 mit den Löchern 22 fluchtet. Der Querschnitt der Drosselbohrungen 42 in einer jeden Reihe 40 ist derselbe, von Reihe 40 zu Reihe 40 aber verschieden, wobei sich der Querschnitt in Umfangsrichtung der Überwurfhülse 38 stetig verändert. Je nach Einstellung der Überwurfhülse 38 wird der Luftaustritt aus dem Steigrohr 18 wohldefiniert gedrosselt, womit eine Anpassung der Anzeige an die unterschiedlichen Lungenvolumina verschiedener Patienten vorgenommen werden kann.

Bei einem Demonstrationsmodell hat der waagrechte Teil des Luftausstoßkanals 12 eine Länge von ca. 15 cm und das Steigrohr eine Länge von ca. 30 cm. Der Rohrdurchmesser beträgt durchweg ca. 2 cm bis 2,5 cm. Die Löcher 22 in der Wand des Steigrohrs 18 haben einen Durchmesser von 2,0 mm bis 2,5 mm, was auch der Durchmesser der größten Drosselbohrungen 42 in der Überwurfhülse 38 ist. Der Abstand der Löcher beträgt ca. 15 mm. Die Löcher 22 des Steigrohrs und/oder der Überwurfhülse 38 können aber in verschiedenen Höhenabschnitten auch einen unterschiedlichen Abstand und/oder unterschiedlichen Durchmesser besitzen, um zum Beispiel im oberen Teil des Steigrohrs 18 eine Nichtlinearisierung der Skala zu erreichen und diese insbesondere disproportional zu verkürzen. Im unteren und mitleren Bereich des Steigrohrs 18 sollte hingegen eine möglichst lineare Beziehung zwischen der Steighöhe des Schwebekörpers 20 und der Ausatmungsströmungsgeschwindigkeit bestehen.

Das in Fig. 5 und 6 dargestellte Atemtherapiegerät hat grundsätzlich denselben Aufbau, wie das zuvor behandelte Atemtherapiegerät. Gleiche Teile sind mit übereinstimmenden Bezugszeichen versehen. Luftansaugkanal 12 und Luftausstoßkanal 14 sind bei dem Atemtherapiegerät in einigem Abstand parallel zueinander angeordnet und über zwei Stutzen 44, 54 miteinander verbunden, wobei der dem Mundstück 10 benachbarte Stutzen 44 einen Durchtrittskanal aufweist, der von einem Kugelventil beherrscht wird. Eine Ventilkugel 46 sitzt passend in einem konischen Ventilsitz 48, der in einem sich über den Querschnitt des Durchtrittskanals erstreckenden Boden 50 ausgebildet ist. Beim Einatmen durch den Luftansaugkanal 12 hebt die Ventilkugel 46 von dem Ventilsitz 48 ab, während sie beim Ausatmen abdichtend damit zur Anlage kommt. Die Abhebebewegung der Ventilkugel 46 ist durch eine als Anschlag wirkende, in die Durchtrittsöffnung hineinragende Lippe 52 begrenzt. Der andere Stutzen 54 ist durch eine Platte 56 blindgeflanscht, die einen Luftdurchtritt durch den Stutzen 54 verhindert. Die aufgelöste Bauweise mit den voneinander beabstandeten Luftansaug- und -ausstoßkanälen 12, 14 erleichtert ein Greifen und Halten des Atemtherapiegeräts.

Das Steigrohr 18 enthält einen diaboloidförmigen Schwebekörper 20. Der Schwebekörper 20 hat einen symmetrischen Aufbau, damit beim Zusammenbau des Atemtherapiegeräts keine Verwechslungen auftreten. Als Ventilglied dient eine Halbkugel 80, die mit dem Ventilsitz 82 zusammenarbeitet. Der Schwebekörper 20 hat zwei umlaufende Dichtnasen bzw. Dichtringe 84, zwischen denen und der Innenwand des Steigrohrs 18 ein Undichtigkeitsspalt 86 besteht. Der diaboloidförmige Schwebekörper 20 zeichnet sich durch geringe Reibung aus. Er verkantet nicht und er ermöglicht minimale Toleranzen zur Abdichtung an der Rohrwand. Weiterhin ist der Aufbau eines beidseitigen Halbkugelventils möglich.

Alternativ zum normalen, oben beschriebenen Atemtherapie-Steigrohr, das eine Beobachtung der kontinuierlichen normalen Atemströmung erlaubt, ist gemäß Fig. 5 an der Stelle des Steigrohrs 18 ein zur Peak-Flow-Messung dienendes Aufsatzrohr 88 an den Luftausstoßkanal 14 angebaut. Dieses dient zum Messen der maximalen Ausatmungsströmung und damit zum Überprüfen der Wirkung von Bronchialdilatoren. Das Peak-Flow-Aufsatzrohr 88 ist nicht geeignet für die Atemtherapiedurchführung und nur bedingt geeignet zum gleichzeitigen Inhalieren aus der Aerosolkammer.

An der Basis des senkrecht zu dem Luftansaug- und -ausstoßkanal orientierten Aufsatzrohrs 88 ist eine Lochplatte 58 eingesetzt, durch deren Öffnungen 60 die Luft in das Aufsatzrohr 88 eintritt. Die Lochplatte 58 dient als Halterung für das Ende einer mittig und axial in dem Aufsatzrohr 88 angebrachten Führungsstange 62, deren anderes Ende in einen das Aufsatzrohr 88 verschlieflenden Deckel 64 eingelassen ist. Auf die Führungsstange 62 ist mit einem zentralen Schaft 66 ein Kolben aufgezogen, der an seinem unteren Ende einen von dem Schaft 66 radial nach außen abstehenden Teller 68 aufweist. Um den Schaft 66 des Kolbens herum liegt eine weiche Schraubendruckfeder 70, die sich einends an dem Teller 68, und andernends an dem Deckel 64 abstützt. Die Feder 70 spannt den Kolben in eine Stellung vor, in der der Teller 68 an der Lochplatte 58 anliegt und deren Löcher verschließt. Beim Ausatmen durch den Luftausstoßkanal 14 wird der Kolben gegen die Kraft der Feder 70 nach oben geblasen.

Das Aufsatzrohr 88 hat eine Undichtigkeit in Gestalt eines sich in Längsrichtung des Aufsatzrohrs 88 erstreckenden Schlitzes 72. Ersichtlich steht ein um so größerer Querschnitt für den Luftdurchtritt durch den Schlitz 72 zur Verfügung, je höher der Kolben 66, 68 in dem Aufsatzrohr 88 steht. An der Oberseite des Kolbentellers 68 liegt lose eine längsgeschlitzte elastische Hülse 74 an, die reibschlüssig in dem Aufsatzrohr 88 geführt ist und durch ihre Eigenelastizität mit soviel Spannung an dem Innenmantel des Aufsatzrohrs 88 anliegt, daß ein Herabrutschen der Hülse 74 allein aufgrund ihres Eigengewichts nicht möglich ist. Beim Hochblasen des Kolbens 66, 68 nimmt dieser die Hülse 74 mit. Letztere bleibt in der höchsten erreichten Stellung des Kolbens 66, 68 hängen, und bildet so eine Anzeige für den maximal gemessenen Volumenstrom der ausgeatmeten Luft. Der Kolben (die Kolbenscheibe) wird durch maximale-stoßweise Ausatmung gegen die Kraft der Feder 70 beschleunigt; je nach Beschleunigung wird das Anzeigeelement auf der Skala nach oben verschoben und zeigt somit den nahezu proportionalen Wert für den maximalen Atemstrom an.

Zum Zurückstellen ist die Hülse 74 mit einem radial davon abstehenden, durch den Schlitz 72 des Aufsatzrohrs 88 nach außen ragenden Griffansatz 76 versehen. Letzterer liegt dem Längsschlitz 78 der Hülse 74 diametral gegenüber. Der Schlitz 72 in dem Aufsatzrohr 88 dient als Führung für den Griffansatz 76, an dem sich die Hülse 74 in Längsrichtung des Aufsatzrohrs 88 verstellen läßt. Der Griffansatz 76 bildet überdies den Zeiger für eine Skala an dem Aufsatzrohr 88.

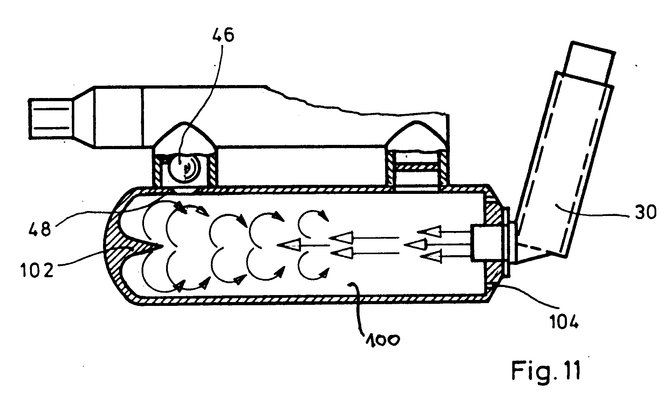

Fig. 11 zeigt ein drittes Atemtherapiegerät, bei der eine große Luftansaugkammer 12 (vgl. auch Fig. 5) als Aerosolverwirbelungskammer mit einer strömungstechnischen Besonderheit ausgebildet ist. An der dem Anschluß für den Dosieraerosolapplikator 30 gegenüberliegenden Seite der Aerosolverwirbelungskammer 100 befindet sich ein Kegel 102 mit einer gerundeten Basis zur Verwirbelung des aus dem Dosieraerosolapplikator 30 austretenden Jet-Strahls. Dem Anschluß des Dosieraerosolapplikators 30 benachbart sind Schlitze bzw. Bohrungen 104 für den Luftrückstrom in der Aerosolverwirbelungskammer 100 ausgebildet. Bei der Füllung der Aerosolkammer 100 mit ein bis zwei Sprühstößen aus dem Dosieraerosolapplikator 30 muß Luft entweichen ohne Verlust von Aerosol. Dies wird erreicht, indem der zentral nach vorne gerichtete "Jetstrahl" des Dosieraerosols auf eine Kegelspitze mit gerundeter Basiskegelfläche trifft und so umgelenkt wird, daß eine Verwirbelung eintritt. Während dieses Vorgangs entweicht die unter Überdruck stehende Luft der Kammer nach hinten durch die mehreren radial angeordneten Bohrungen 104 um den Ansatz des Dosieraerosolapplikators 30 herum. Ein Verlust über das Kugelventil zum Mundstück hin kann vernachlässigt werden (Proportion Bohrung zur übrigen Ringfläche im Bereich der Bohrung = 1/20 bei einem Rohrdurchmesser von 60 mm und einer Bohrung des Ventil von ca. 10 mm Durchmesser).

Das erfindungsgemäße Atemtherapiegerät zeichnet sich durch einen sehr geringen Totraum im Mundstückbereich 10 aus, der bei der normalen Ausatmung unter Einatumung von Frischluft zu vernachlässigen ist. Beim Einatmen gelangt daher fast keine verbrauchte Luft in die Lunge. Durch die Ausatmung gegen den Widerstand, den das Steigrohr mit dem darin geführten Schwebekörper bietet, wird ein gleichmäßiges Ausatmen gefördert. Dank der sehr nahen Position des Ventils zum Mundstück erfolgt die Inhalation ohne wesentliche Rückatmung von Totraumluft. Bei der Therapie mit einem Dosieraerosol bildet der Luftansaugkanal eine Aerosolkammer, in die das Dosieraerosol eingeleitet werden kann, ohne daß der am Steigrohr 18 beobachtete Ausatemvorgang unterbrochen oder in seinem Ablauf beeinflußt werden müßte. Es versteht sich, daß das Volumen der Aerosolkammer bei Bedarf auch größer gewählt, und ein Anschluß von verschiedenen Inhalationsgeräten über geeignete Adapter erfolgen kann. Die Einatmung des Aerosols erfolgt über ein unterdruckgesteuertes Ventil, das dank der Baugeometrie des Atemtherapiegeräts durch den Sprühstoß des Aerosols nicht geöffnet werden kann. Durch den Aerosolverwirbelungskegel wird eine Verlangsamung der Aerosolströmung und eine vorteilhafte Verwirbelung und Vermischung des Aerosols erreicht. Die Kondensatablagerung ist proportional jur Kammerinnenfläche, weshalb sich eine kleinvolumige Auslegung der Inhalationskammer empfiehlt, die noch eine Füllung mit ein bis zwei Dosieraerosolsprühstößen zuläßt (ohne Verlust durch Rückströmung).

## Patentansprüche

1. Atemtherapiegerät für an Asthma oder spastischer Bronchitis leidende Personen mit einem Gehäuse, zu dem ein Mundstück (10), ein Anschluß für einen Inhalationsmittelapplikator (30) und ein Luftausstoßkanal (14) gehören und das eine atemstrombetätigte Ventilanordnung enthält, die beim Einatmen einen Strömungsweg von dem Mundstück (10) in das Gehäuse freigibt und beim Ausatmen sperrt sowie beim Einatmen den Luftausstoßkanal (14) sperrt und beim Ausatmen freigibt, dadurch gekennzeichnet, daß zu dem Gehäuse eine große Aerosolverwirbelungskammer mit dem Anschluß für den Inhalationsmittelapplikator (30) und mit einem diesem Anschluß gegenüberliegenden Verwirbelungskegel (102) gehört, und daß der Luftausstoßkanal über eine Meß- und Anzeigevorrichtung für den Volumenstrom der ausgeatmeten Luft zur Atmosphäre führt, die ein Steigrohr (18) und einen darin passend aufgenommenen Schwebekörper (20) aufweist.

2. Atemtherapiegerät nach Anspruch 1, dadurch gekennzeichnet, daß das Steigrohr (18) einen Luftdurchtritt mit einem Strömungswiderstand ermöglicht, der mit zunehmender Höhe des Schwebekörpers (20) abfällt.

3. Atemtherapiegerät nach Anspruch 1 oder 2, dadurch gekennzeichnet, daß über wenigstens einen Teil der Steigrohrlänge eine lineare Beziehung zwischen der Höhe des Schwebekörpers (20) und dem Volumenstrom der austretenden Luft besteht.

4. Atemtherapiegerät nach einem der Ansprüche 1 bis 3, dadurch gekennzeichnet, daß der Schwebekörper als Freikolben (20) gestaltet ist, z. B. als "Diaboloid".

5. Atemtherapiegerät nach einem der Ansprüche 1 bis 4, dadurch gekennzeichnet, daß der Schwebekörper (20) als Ventilglied fungiert und in seiner tiefsten Position das Steigrohr (18) an einem Ventilsitz (82) dicht verschließt.

6. Atemtherapiegerät nach einem der Ansprüche 1 bis 5, dadurch gekennzeichnet, daß die Wand des Steigrohrs (18) eine Undichtigkeit aufweist.

7. Atemtherapiegerät nach einem der Ansprüche 1 bis 6, dadurch gekennzeichnet, daß die Wand des Steigrohrs (18) mit einer Anzahl vorzugsweise in einer Reihe höhenversetzt angeordneter Löcher (22) versehen ist.

8. Atemtherapiegerät nach einem der Ansprüche 1 bis 7, dadurch gekennzeichnet, daß die Wand des Steigrohrs (18) mit einem vorzugsweise in Längsrichtung sich erstreckenden Schlitz versehen ist.

9. Atemtherapiegerät nach einem der Ansprüche 1 bis 8, dadurch gekennzeichnet, daß eine verstellbare Abdeckung vorgesehen ist, mit der sich die Undichtigkeit zum Teil verschließen läßt.

10. Atemtherapiegerät nach einem der Ansprüche 1 bis 9, dadurch gekennzeichnet, daß die Abdeckung von einer drehbar auf das Steigrohr (18) aufgezogenen Überwurfhülse (32, 38) gebildet ist.

11. Atemtherapiegerät nach einem der Ansprüche 1 bis 10, dadurch gekennzeichnet, daß die Überwurfhülse (32) einen Ausschnitt (34) hat, dank dessen sie je nach Winkelstellung eine mehr oder weniger große Anzahl von Löchern (22) abdeckt.

12. Atemtherapiegerät nach einem der Ansprüche 1 bis 11, dadurch gekennzeichnet, daß die Überwurfhülse (38) mehrere Reihen (40) reihenweise vorzugsweise gleich großer, von Reihe zu Reihe verschieden großer Drosselbohrungen (42) hat, die je nach Winkelstellung der Überwurfhülse (38) mit der Lochreihe (22) des Steigrohrs (18) fluchten.

13. Atemtherapiegerät nach einem der Ansprüche 1 bis 12, dadurch gekennzeichnet, daß das Steigrohr (18) unter einem Winkel zwischen 0° und 20°, vorzugsweise ca. 10°, gegen die Vertikale angestellt ist.

14. Atemtherapiegerät nach einem der Ansprüche 1 bis 13, dadurch gekennzeichnet, daß das Steigrohr (18) lösbar mit dem Gehäuse verbunden und gegen ein zur Peak-Flow-Messung dienendes Aufsatzrohr (88) austauschbar ist.

15. Atemtherapiegerät nach einem der Ansprüche 1 bis 14, dadurch gekennzeichnet, daß das Aufsatzrohr (88) einen Kolben (66, 68) enthält, und daß in dem Aufsatzrohr (88) ein selbsthemmend daran festliegendes, von dem Kolben mitgenommenes Anzeigeelement reibschlüssig geführt ist.

16. Atemtherapiegerät nach einem der Ansprüche 1 bis 15, dadurch gekennzeichnet, daß der Kolben (66, 68) auf eine Führungsstange (62) aufgezogen ist.

17. Atemtherapiegerät nach einem der Ansprüche 1 bis 16, dadurch gekennzeichnet, daß der Kolben (66, 68) durch eine Feder (70) niedergespannt ist.

18. Atemtherapiegerät nach einem der Ansprüche 1 bis 17, dadurch gekennzeichnet, daß das Anzeigeelement eine längsgeschlitzte (78) elastische Hülse (74) ist.

19. Atemtherapiegerät nach einem der Ansprüche 1 bis 18, dadurch gekennzeichnet, daß das Anzeigeelement mit einem durch einen Schlitz (72) in der Wand des Aufsatzrohres (88) hindurchragenden Griffansatz (76) versehen ist.

## Claims

1. Respiratory therapy device for persons suffering from asthma or bronchospasm, with a housing having a mouthpiece (10), a connection for an inhalant applicator (30) and an air expulsion channel (14) and containing a respiratory flow-actuated valve arrangement which on inhalation clears, and on exhalation blocks a flow path from the mouthpiece (10) into the housing as well as on inhalation blocks and on exhalation clears the air expulsion channel (14), characterized in that the housing has a large aerosol vortexing chamber with a connection for the inhalant applicator (30) and a vortexing cone (102) lying opposite said connection, and that the air expulsion channel leads to atmosphere via an apparatus, which measures and displays the volumetric rate of flow of the exhaled air and has an upright tube (18) with a suspended body (20) fitted therein.

2. Respiratory therapy device according to claim 1, characterized in that the upright tube (18) allows a passage of air with a flow resistance which diminishes as the level of the suspended body (20) rises.

3. Respiratory therapy device according to claim 1 or 2, characterized in that over at least a portion of the upright tube length there is a linear relationship between the level of the suspended body (20) and the volumetric rate of flow of the outgoing air.

4. Respiratory therapy device according to one of claims 1 to 3, characterized in that the suspended body is designed as a free piston (20), e.g. as an element shaped like a two-headed top.

5. Respiratory therapy device according to one of claims 1 to 4, characterized in that the suspended body (20) acts as a valve member and in its lowest position tightly closes the upright tube (18) at a valve seat (82).

6. Respiratory therapy device according to one of claims 1 to 5, characterized in that the wall of the upright tube (18) has a leakage point.

7. Respiratory therapy device according to one of claims 1 to 6, characterized in that the wall of the upright tube (18) is provided with a number of holes (22) which are preferably arranged vertically offset in a row.

8. Respiratory therapy device according to one of claims 1 to 7, characterized in that the wall of the upright tube (18) is provided with a slot which preferably extends in a longitudinal direction.

9. Respiratory therapy device according to one of claims 1 to 8, characterized in that an adjustable covering is provided, by means of which the leakage point may be partially closed.

10. Respiratory therapy device according to one of claims 1 to 9, characterized in that the covering is formed by a wrapping sleeve (32, 38) drawn rotatably onto the upright tube (18).

11. Respiratory therapy device according to one of claims 1 to 10, characterized in that the wrapping sleeve (32) has a cutout (34) by means of which the sleeve, depending on its angular position, covers a greater or smaller number of holes (22) .

12. Respiratory therapy device according to one of claims 1 to 11, characterized in that the wrapping sleeve (38) has a plurality of rows (40) of throttle bores (42) whose size is identical within a row but differs from row to row and which, depending on the angular position of the wrapping sleeve (38), are in alignment with the row of holes (22) of the upright tube (18).

13. Respiratory therapy device according to one of claims 1 to 12, characterized in that the upright tube (18) is set at an angle of between 0° and 20°, preferably around 10°, relative to vertical.

14. Respiratory therapy device according to one of claims 1 to 13, characterized in that the upright tube (18) is detachably connected to the housing and is exchangeable for an attachment tube (88) used for peak flow measurement.

15. Respiratory therapy device according to one of claims 1 to 14, characterized in that the attachment tube (88) includes a piston (66, 68), and that guided in a frictionally engaged manner in the attachment tube (88) is a display element which is fixed in a self-locking manner against said tube and is driven by the piston.

16. Respiratory therapy device according to one of claims 1 to 15, characterized in that the piston (66, 68) is drawn up onto a guide rod (62).

17. Respiratory therapy device according to one of claims 1 to 16, characterized in that the piston (66, 68) is stressed downwards by a spring (70).

18. Respiratory therapy device according to one of claims 1 to 17, characterized in that the display element is an elastic sleeve (74) with a longitudinal slit (78).

19. Respiratory therapy device according to one of claims 1 to 18, characterized in that the display element is provided with a grip attachment (76) which projects through a slot (72) in the wall of the attachment tube (88).

## Revendications

1. Appareil de thérapie respiratoire pour personnes souffrant d'asthme ou de bronchite spasmodique, comportant un boîtier dont font partie un embout (10), un raccord pour un applicateur de produit d'inhalation (30) ainsi qu'un conduit d'expulsion d'air (14) et qui contient une disposition de soupapes actionnées par le flux respiratoire, qui, à l'inspiration, ouvre un parcours d'écoulement menant de l'embout (10) au boîtier et qui ferme le conduit d'expulsion d'air (14) à l'expiration comme à l'inspiration et l'ouvre à l'expiration, caractérisé en ce que le boîtier comprend une grande chambre de mise en tourbillonnement d'un aérosol avec le raccord pour l'applicateur de produit d'inhalation (30) ainsi qu'un cône de tourbillonnement (102), opposé à ce raccord et en ce que le conduit d'expulsion d'air mène à l'atmosphère par l'intermédiaire d'un dispositif de mesure et d'affichage du flux volumique de l'air expiré, qui comporte un tube ascendant (18) et un corps flottant (20), logé ajusté dans celui-ci.

2. Appareil de thérapie respiratoire selon la revendication 1, caractérisé en ce que le tube ascendant (18) permet un passage d'air avec une perte de charge qui diminue lorsqu'augmente la hauteur du corps flottant (20).

3. Appareil de thérapie respiratoire selon la revendication 1 ou 2, caractérisé en ce qu'il existe une relation linéaire entre la hauteur du corps flottant (20) et le flux volumique de l'air expulsé, sur au moins une partie de la longueur du tube ascendant.

4. Appareil de thérapie respiratoire selon l'une des revendications 1 à 3, caractérisé en ce que le corps flottant a la forme d'un piston libre (20), par exemple d'un "diabolo".

5. Appareil de thérapie respiratoire selon l'une des revendication. 1 à 4, caractérisé en ce que le corps flottant (20) sert d'organe de soupape et dans sa position la plus basse ferme hermétiquement le tube ascendant (18) contre un siège de soupape (82).

6. Appareil de thérapie respiratoire selon l'une des revendications 1 à 5, caractérisé en ce que la paroi du tube ascendant (18) présente un défaut d'étanchéité.

7. Appareil de thérapie respiratoire selon l'une des revendications 1 à 6, caractérisé en ce que la paroi du tube ascendant (18) est pourvue d'un certain nombre de trous (22), de préférence disposés en une rangée, décalés en hauteur.

8. Appareil de thérapie respiratoire selon l'une des revendications 1 à 7, caractérisé en ce que la paroi du tube ascendant (18) est pourvue d'une fente, s'étendant de préférence dans la direction longitudinale.

9. Appareil de thérapie respiratoire selon l'une des revendications 1 à 8, caractérisé en ce qu'il est prévu une couverture réglable permettant de fermer en partie le défaut d'étanchéité.

10. Appareil de thérapie respiratoire selon l'une des revendications 1 à 9, caractérisé en ce que la couverture est formée par un manchon formant chapeau (32, 38), enfilé de manière à pouvoir tourner sur le tube ascendant (18).

11. Appareil de thérapie respiratoire selon l'une des revendications 1 à 10, caractérisé en ce que le manchon (32) possède une découpe (34) lui permettant de recouvrir un nombre plus ou moins grand de trous (22), suivant sa position angulaire.

12. Appareil de thérapie respiratoire selon l'une des revendications 1 à 11, caractérisé en ce que le manchon (38) possède plusieurs rangées (40) d'alésages d'étranglement (42) de préférence de mêmes dimensions dans une même rangée et de dimensions différentes d'une rangée à l'autre, qui sont alignés avec la rangée de trous (22) du tube ascendant (18), suivant la position angulaire du manchon (38).

13. Appareil de thérapie respiratoire selon l'une des revendications 1 à 12, caractérisé en ce que le tube ascendant (18) est incliné par rapport à la verticale, d'un angle compris entre 0° et 20°, de préférence égal à 10° environ.

14. Appareil de thérapie respiratoire selon l'une des revendications 1 à 13, caractérisé en ce que le tube ascendant (18) est assemblé de manière amovible avec le boîtier et peut être substitué par un tube rapporté (88), servant à mesurer le flux de pointe.

15. Appareil de thérapie respiratoire selon l'une des revendications 1 à 14, caractérise en ce que le tube rapporté (88) contient un piston (66, 68) et en ce que dans le tube rapporté (88) est guidé, par friction, un élément d'affichage, entraîné par le piston, et fixé sur ce dernier de manière autobloquante.

16. Appareil de thérapie respiratoire selon l'une des revendications 1 à 15, caractérisé en ce que le piston (66, 68) est emmanché sur une tige de guidage (62).

17. Appareil de thérapie respiratoire selon l'une des revendications 1 à 16, caractérisé en ce que le piston (66, 68) est pressé par un ressort (70).

18. Appareil de thérapie respiratoire selon l'une des revendications 1 à 17, caractérisé en ce que l'élément d'affichage est une gaine (74), élastique fendue longitudinalement.

19. Appareil de thérapie respiratoire selon l'une des revendications 1 à 18, caractérisé en ce que l'élément d'affichage est pourvu d'une poignée (76) traversant la paroi du tube rapporté (88).
